# EUROPEAN PATENT SPECIFICATION

(11) **EP 0 965 352 B1**
(45) Date of publication and mention of the grant of the patent: **27.11.2002**
(21) Application number: 99303814.0
(22) Date of filing: 17.05.1999
(51) Int. Cl.: A61L 2/06

(54) **Autoclaves**
Autoklaven
Autoclaves

(30) Priority: 16.06.1998 GB 9812878
(43) Date of publication of application: 22.12.1999
(73) Proprietor: Eschmann Holdings Limited, Lancing, West Sussex BN15 8TJ (GB)
(72) Inventor: Hannant, Keith, Rustington, West Sussex BN16 2QN (GB)
(74) Representative: Jenkins, Peter David

(56) References cited:
- US-A- 3 410 650
- US-A- 3 826 612
- US-A- 3 884 636
- US-A- 5 103 076

## Description

This invention relates to autoclaves of the kind having an autoclave chamber, a water reservoir arranged to supply water to the chamber and an outer housing.

Autoclaves are used to treat articles with steam at elevated temperature and pressure, such as to effect sterilization. Water is supplied to the autoclave chamber at the start of each treatment cycle from a reservoir enclosed within the autoclave housing, the water being heated in the chamber to produce the steam. At the end of the cycle, water may be returned to the reservoir. A certain amount of water is lost each treatment cycle and it is necessary periodically to top up the reservoir. Larger chambers required larger water reservoirs, thereby increasing the overall size of the autoclave. Where the size of the autoclave must be kept to a minimum, this can mean that the reservoir is smaller than desirable, requiring frequent refilling. This problem is made worse by the need for the reservoir to fit around the other components in the autoclave housing.

US-A-5,103,076 discloses a double steam oven having a heating or sterilisation chamber defined by an inner oven wall, and an outer oven wall surrounding and spaced from the inner oven wall. A hot water tank is provided at the bottom of the inner oven where steam is generated by a suitable heater. Steam rises through the inner oven and exits therefrom at the top wall into the outer oven, and is then condensed to form water which is returned to the water tank.

US-A-3,410,650 discloses an autoclave comprising a pressure chamber having a heating element adjacent the lower wall of the chamber for heating water in the bottom of the autoclave. Steam leaves the pressure chamber via a capillary tube, which passes through a condensing chamber, with the tube having an end located outside the autoclave for delivering condensed steam into a beaker.

It is an object of the present invention to provide an improved autoclave.

According to the present invention there is provided an autoclave having an autoclave pressure chamber, a water reservoir arranged to supply water to the chamber and an outer housing, wherein the water reservoir comprises a molding defining a hollow panel-shape compartment which provides a wall of the outer housing and the water reservoir communicates with the autoclave pressure chamber via a pipe and valve.

The panel-shape compartment preferably extends across the width of the housing. The autoclave chamber preferably opens at the front of the autoclave, the reservoir providing a rear wall of the housing. An outer wall of the reservoir may have a plurality of stand-off pillars adapted to space the wall of the autoclave from external objects. The reservoir may have at least one air vent aperture extending through the reservoir to allow air to flow into or out of the housing. The reservoir preferably includes a filling tube extending from the reservoir to the front of the autoclave. The reservoir may have a front face with a concave recess, the rear end of the autoclave chamber being received in the recess. The reservoir may have a box formation projecting from a front face and projecting alongside the autoclave chamber. The reservoir is preferably a single-piece moulding of a plastics material.

An autoclave according to the present invention, will now be described, by way of example, with reference to the accompanying drawings, in which:

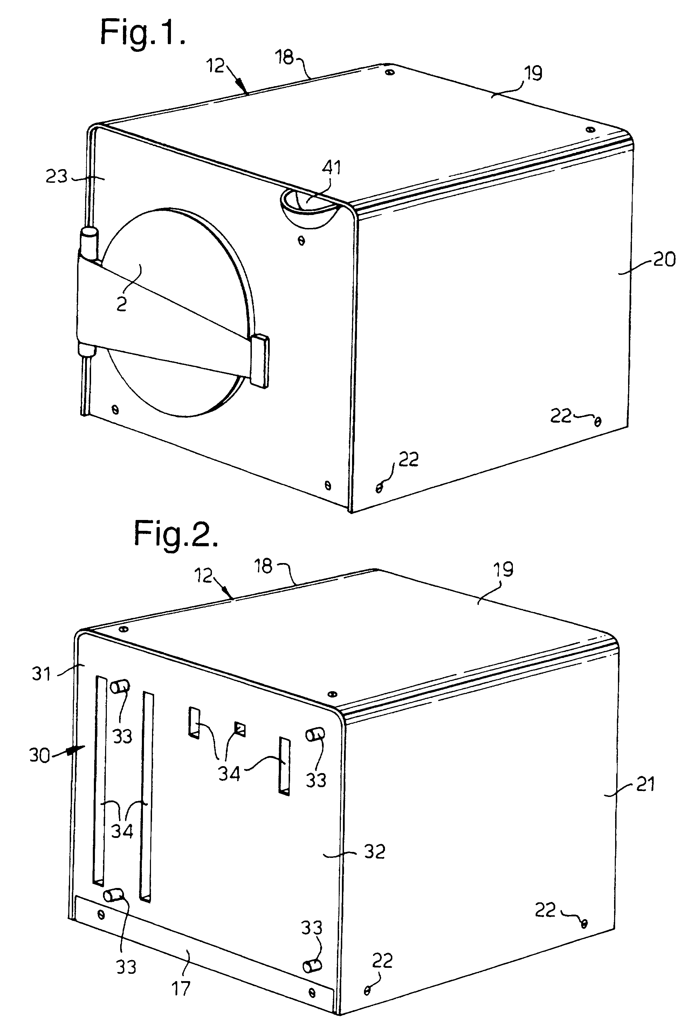

- Figure 1: is a perspective view of the front of the autoclave;
- Figure 2: is a perspective view of the rear of the autoclave;

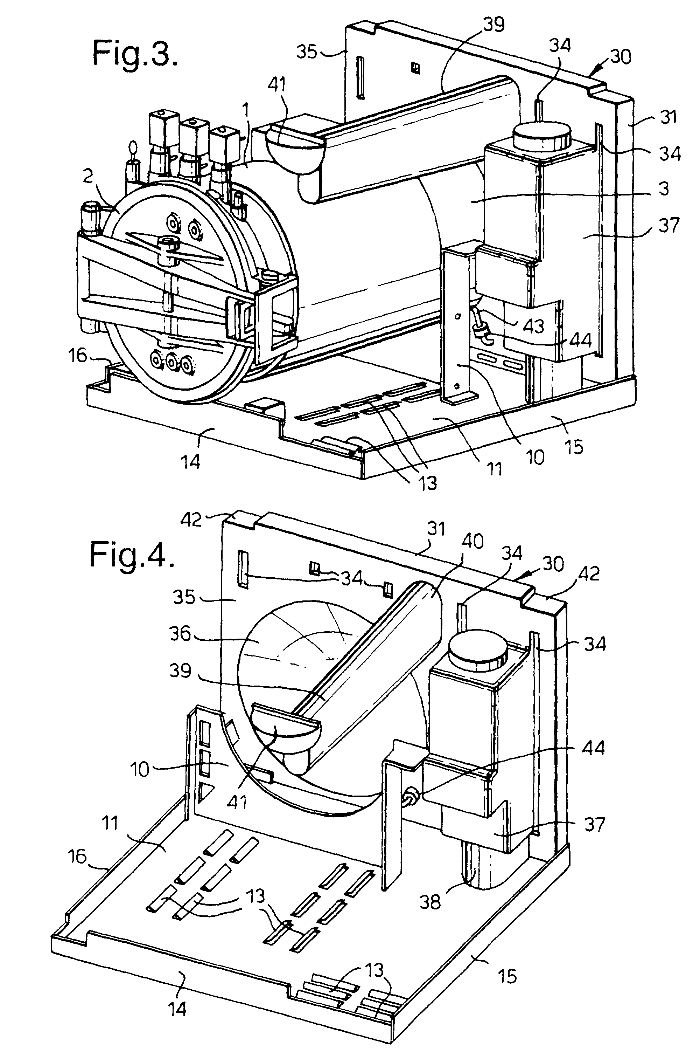

- Figure 3: is a perspective, simplified view of the front of the autoclave with a part of its housing removed; and
- Figure 4: is a perspective, simplified view of the floor of the autoclave with the autoclave chamber removed.

The autoclave has a pressure chamber 1 of cylindrical shape with a door 2 at one end and a convex, domed end wall (not shown) at its rear end 3. The chamber 1 is supported on a bracket 10 attached with a floor panel 11. The floor panel 11 provides a part of the autoclave housing 12, the panel being a pressed metal sheet with air vents 13 and with turned-up edges 14 to 17 around all four sides. The housing 12 contains the-conventional components of an autoclave, not shown here, for clarity.

The housing 12 also includes a U-shape bent metal cover 18 forming the top 19 and two sides 20 and 21. The cover 18 is secured to the floor panel 11 by screws 22 engaging the edges 15 and 16 of the floor panel. A flat, metal sheet front panel 23 is secured at the front of the housing 12.

The autoclave includes a water reservoir 30 made as a single-piece rotational moulding of a plastics material, such as polythene or polypropylene, although it could be formed in several parts. The reservoir 30 includes a square, hollow panel-shape compartment 31, which provides the rear wall of the housing 12 and extends across the entire width and height of the autoclave. The rear wall 32 of the compartment 31 is exposed at the rear of the autoclave and has several stand-off pillars 33 so as to ensure that the rear of the autoclave is not placed too close to external objects, which might otherwise restrict air flow around the unit. The compartment 31 has several air vent apertures in the form of slots 34 formed through it to allow air to flow through the compartment, into or out of the interior of the housing 12. On its front face 35, the compartment 31 has a concave, domed recess 36 towards its left-hand side, which is located to receive the rear end 3 of the autoclave chamber 1. The reservoir 30 also includes a hollow box formation 37 of generally rectangular shape projecting from the front face 35 of the compartment 31, the box being shaped to fit around other components of the autoclave within the housing. In particular, the box 37 extends alongside the autoclave chamber 1, the box having a foot 38 that sits on the upper surface of the floor panel 11. The water reservoir 30 further includes an integral filling spout 39 in the form of a substantially horizontal hollow tube 40 that extends forwardly and is terminated at its forward end by a filling cup 41 positioned on the front panel 23. The interior of the spout 39, the compartment 31 and the box 37 form a common, intercommunicating cavity so that water poured in the cup 41 will flow to fill the entire reservoir 30. The moulding of the reservoir 30 is formed with various screw holes 42 or other fixings so that it can be attached to the floor panel 11 and the cover 18. The interior of the reservoir 30 also communicates with the interior of the autoclave chamber via a pipe 43 and valve 44.

The water reservoir 30, therefore, serves an additional purpose of providing one of the walls of the autoclave housing 12. This enables a relatively large volume reservoir to be used whilst allowing a simple layout of the interior of the autoclave. Because a wall 32 of the reservoir 30 is exposed on the outside of the autoclave, it helps dissipate heat from water returned to the reservoir from the chamber 1 after a treatment cycle. Dissipation of heat is improved by the large surface area of the panel-shape compartment 31, which is further increased by the air vents 34 through the compartment.

## Claims

1. An autoclave having an autoclave pressure chamber (1), a water reservoir (30) arranged to supply water to the chamber and an outer housing (12), wherein the water reservoir (30) comprises a molding defining a hollow panel-shape compartment which provides a wall (32) of the outer housing (12) and the water reservoir (30) communicates with the autoclave pressure chamber (1) via a pipe (43) and valve (44).

2. An autoclave according to claim 1, wherein the panel-shape compartment (31) extends across the width of the housing (12).

3. An autoclave according to claim 1 or 2, wherein the autoclave chamber (1) opens at the front of the autoclave, and the reservoir (30) provides a rear wall (32) of the housing (12).

4. An autoclave according to any one of the preceding claims, wherein an outer wall (32) of the reservoir (30) has a plurality of stand-off pillars (33) adapted to space the wall (32) of the autoclave from external objects.

5. An autoclave according to any one of the preceding claims, wherein the reservoir (30) has at least one air vent aperture (34) extending through the reservoir to allow air to flow into or out of the housing (12).

6. An autoclave according to any one of the preceding claims, wherein the reservoir (30) includes a filling tube (40) extending from the reservoir to the front of the autoclave.

7. An autoclave according to any one of the preceding claims, wherein the reservoir (30) has a front face (35) with a concave recess (36), and the rear end (3) of the autoclave chamber (1) is received in the recess (36).

8. An autoclave according to any one of the preceding claims, wherein the reservoir (30) has a box formation (37) projecting from the front face (35) and projecting alongside the autoclave chamber (1).

9. An autoclave according to any one of the preceding claims, wherein the reservoir (30) is a single-piece moulding of a plastics material.

## Patentansprüche

1. Autoklav mit einer Autoklav-Druckkammer (1), einem Wasserbehälter (30), der angeordnet ist, um Wasser zu der Kammer zu führen, und einem äußeren Gehäuse (12), wobei der Wasserbehälter (30) ein Formteil umfasst, das ein hohles, plattenförmiges Behältnis festlegt, das eine Wand (32) des äußeren Gehäuses (12) bereit stellt, und der Wasserbehälter (30) mit der Autoklav-Druckkammer (1) über ein Rohr (43) und ein Ventil (44) in Verbindung steht.

2. Autoklav nach Anspruch 1, bei welchem das plattenförmige Behältnis (31) sich über die Breite des Gehäuses (12) erstreckt.

3. Autoklav nach Anspruch 1 oder 2, bei welchem die Autoklav-Kammer (1) auf der Vorderseite des Autoklavs aufgeht und der Behälter (30) eine Rückwand (32) des Gehäuses (12) bereit stellt.

4. Autoklav nach einem der vorhergehenden Ansprüche, bei welchem eine äußere Wand (32) des Behälters (30) eine Vielzahl von abstehenden Säulen bzw. Stützen (33) hat, die angepasst sind, die Wand (32) des Autoklavs von externen Objekten zu beabstanden.

5. Autoklav nach einem der vorhergehenden Ansprüche, bei welchem der Behälter (30) wenigstens eine Entlüftungsöffnung (34) hat, die sich durch den Behälter erstreckt, um Luft zu ermöglichen, in das Gehäuse (12) zu fließen oder daraus heraus.

6. Autoklav nach einem der vorhergehenden Ansprüche, bei welchem der Behälter (30) eine Füllröhre bzw. einen Füllschlauch (40) enthält, der sich von dem Behälter zu der Vorderseite des Autoklavs erstreckt.

7. Autoklav nach einem der vorhergehenden Ansprüche, bei welchem der Behälter (30) eine Vorderseite (35) mit einer konkaven Vertiefung (36) hat und das rückwärtige Ende (3) der Autoklav-Kammer (1) in der Vertiefung (36) aufgenommen ist.

8. Autoklav nach einem der vorhergehenden Ansprüche, bei welchem der Behälter (30) eine Schachtelform (37) hat, die von der Vorderfläche bzw. Vorderseite (35) vorsteht und entlang der Autoklav-Kammer (1) vorsteht.

9. Autoklav nach einem der vorhergehenden Ansprüche, bei welchem der Behälter (30) ein einstückiges Formteil aus Kunststoffmaterial ist.

## Revendications

1. Autoclave ayant une chambre (1) à pression d'autoclave, un réservoir d'eau (30) agencé pour alimenter en eau la chambre et un corps extérieur (12), dans lequel le réservoir d'eau (30) comporte une pièce moulée définissant un compartiment en forme de panneau creux qui constitue une paroi (32) du corps extérieur (12) et le réservoir d'eau (30) communique avec la chambre (1) de pression de l'autoclave par un conduit (43) et un robinet (44).

2. Autoclave selon la revendication 1, dans lequel le compartiment (31) en forme de panneau s'étend sur la largeur du corps (12).

3. Autoclave selon la revendication 1 ou 2, dans lequel la chambre (1) de l'autoclave s'ouvre à l'avant de l'autoclave, et le réservoir (30) forme une paroi arrière (32) du corps (12).

4. Autoclave selon l'une quelconque des revendications précédentes, dans lequel une paroi extérieure (32) du réservoir (30) comporte plusieurs tiges d'écartement (33) conçues pour espacer la paroi (32) de l'autoclave d'objets extérieurs.

5. Autoclave selon l'une quelconque des revendications précédentes, dans lequel le réservoir (30) présente au moins une ouverture (34) de mise à l'air s'étendant à travers le réservoir pour permettre à de l'air d'entrer dans le corps (12) ou d'en sortir.

6. Autoclave selon l'une quelconque des revendications précédentes, dans lequel le réservoir (30) comprend un tube de remplissage (40) s'étendant depuis le réservoir jusqu'à l'avant de l'autoclave.

7. Autoclave selon l'une quelconque des revendications précédentes, dans lequel le réservoir (30) comporte une face avant (35) présentant un évidement concave (36), et l'extrémité arrière (3) de la chambre (1) de l'autoclave est reçue dans l'évidement (36).

8. Autoclave selon l'une quelconque des revendications précédentes, dans lequel le réservoir (30) comporte une conformation en boîte (37) faisant saillie de la face avant (35) et faisant saillie le long d'un côté de la chambre (1) de l'autoclave.

9. Autoclave selon l'une quelconque des revendications précédentes, dans lequel le réservoir (30) est moulé d'une seule pièce en matière plastique.
